(19) Europäisches Patentamt
European Patent Office
Office européen des brevets

(11) Veröffentlichungsnummer: **0 110 829 B1**

(12) # EUROPÄISCHE PATENTSCHRIFT

(45) Veröffentlichungstag der Patentschrift: **27.12.90**

(51) Int. Cl.$^5$: **A 01 N 43/80,** C 07 D 275/06

(21) Anmeldenummer: **83810537.7**

(22) Anmeldetag: **21.11.83**

(54) **Schädlingsbekämpfungsmittel.**

(30) Priorität: **26.11.82 CH 6893/82
12.10.83 CH 5568/83**

(43) Veröffentlichungstag der Anmeldung:
**13.06.84 Patentblatt 84/24**

(45) Bekanntmachung des Hinweises auf die Patenterteilung:
**27.12.90 Patentblatt 90/52**

(84) Benannte Vertragsstaaten:
**AT BE CH DE FR GB IT LI NL**

(56) Entgegenhaltungen:
**EP-A-0 033 984
DE-A-1 670 921**

**Chemical Abstracts, Band 29, Nr. 12, 20.06.1935, Spalte 3998-9 - 3999-4. Columbus Ohio US; A. Mannessier-Mameli,:"The pyrolysis of saccharin oxime."**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG
Klybeckstrasse 141
CH-4002 Basel (CH)**

(72) Erfinder: **Drabek, Jozef, Dr.
Benkenstrasse 12
CH-4104 Oberwil (CH)**

(56) References cited:
**CHEMICAL ABSTRACTS, Band 90, Nr.19 07-05-1979, Seite 161, Zusammenfassung 146950q. COLUMBUS, OHIO (US) A. BELLOTTI et al.: "Phytotoxicity of 3-acylamino derivatives of 1, 2-benzisothiazole."**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 110 829 B1

Courier Press, Leamington Spa, England.

## Beschreibung

Die vorliegende Erfindung betrifft die Verwendung von 3-Acylaminobenzisothiazol-S,S-dioxiden zur Bekämpfung von Insekten und Vertretern der Ordnung Akarina sowie neue substituierte 3-Acylaminobenzisothiazol-S,S-dioxide, Verfahren zu ihrer Herstellung und Mittel die diese Verbindungen als aktive Komponente enthalten.

Ein Gegenstand der Erfindung ist die Verwendung von 3-Acylaminobenzisothiazol-S,S-dioxiden der Formel I

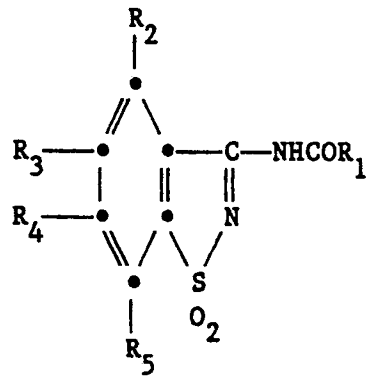

(I) ,

worin $R_1$ $C_1$—$C_{10}$-Alkyl, Phenyl oder durch Halogen oder $C_1$—$C_5$-Alkoxy substituiertes $C_1$—$C_{10}$-Alkyl; und $R_2$, $R_3$, $R_4$ und $R_5$ unabhängig voneinander Wasserstoff, Halogen, $C_1$—$C_5$-Alkyl, $C_1$—$C_5$-Alkoxy, Trifluormethyl, Amino oder Nitro bedeuten, zur Bekämpfung von Insekten und Vertretern der Ordnung Akarina.

Die Verbindungen der Formel I können im Rahmen der vorliegenden Erfindung auch in ihrer tautomeren Form der folgenden Formel Ia

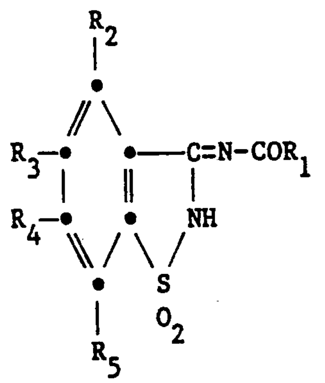

(Ia)

vorliegen.

Bevorzugt werden Verbindungen der Formel I verwendet, worin $R_1$ $C_1$—$C_{10}$-Alkyl, Phenyl oder durch Fluor, Chlor, Brom oder $C_1$—$C_5$-Alkoxy substituiertes $C_1$—$C_{10}$-Alkyl; und $R_2$, $R_3$, $R_4$ und $R_5$ unabhängig voneinander Wasserstoff, Fluor, Chlor, Brom, $C_1$—$C_5$-Alkyl, $C_1$—$C_5$-Alkoxy, Trifluormethyl, Amino oder Nitro bedeuten, insbesondere solche Verbindungen der Formel I, die dadurch gekennzeichnet sind, dass $R_1$ $C_1$—$C_5$-Alkyl, vorzugsweise Methyl, Phenyl oder durch 1 bis 7 Fluor- oder Chloratome substituiertes $C_1$—$C_3$-Alkyl; $R_2$ Fluor, Chlor, Brom, $C_1$—$C_5$-Alkyl, $C_1$—$C_5$-Alkoxy oder Trifluormethyl und $R_3$, $R_4$ und $R_5$ unabhängig voneinander Wasserstoff, Fluor, Chlor, Brom, $C_1$—$C_3$-Alkyl, Methoxy oder Trifluormethyl bedeuten.

Wegen ihrer insektiziden und akariziden Wirkung sind vor allem diejenigen Verbindungen der Formel I hervorzuheben, worin $R_2$ Chlor, $R_3$ Wasserstoff oder Chlor, und $R_4$ und $R_5$ Wasserstoff bedeuten.

In den vorstehend genannten Verbindungen der Formel I ist unter Halogen Fluor, Chlor, Brom oder Jod, insbesondere aber Chlor oder Fluor, zu verstehen. Die bei $R_1$ bis $R_5$ in Frage kommenden Alkyl- und Alkoxygruppen können geradkettig oder verzweigt sein. Beispiele von Alkoxy- und gegebenenfalls substituierten Alkylgruppen bei $R_1$ bis $R_5$ sind u.a.: Methyl, Methoxy, —$CH_2Cl$, Trifluormethyl, Aethyl, Aethoxy, —$CH_2CH_2F$, —$CF_2CF_3$, Propyl, —$CF_2$—$CF_2$—$CF_3$, Isopropyl, n-, i-, sek.-, tert.-Butyl, n-Pentyl, n-Hexyl, n-Decyl und deren Isomere.

Ein weiterer Gegenstand der Erfindung sind neue Verbindungen der Formel I, die dadurch gekennzeichnet sind, dass $R_1$ $C_1$—$C_{10}$-Alkyl, Phenyl oder durch Halogen oder $C_1$—$C_5$-Alkoxy substituiertes $C_1$—$C_{10}$-Alkyl; und $R_2$, $R_3$, $R_4$ und $R_5$ unabhängig voneinander Wasserstoff, Halogen, $C_1$—$C_5$-Alkyl, $C_1$—$C_5$-Alkoxy, Trifluormethyl, Amino oder Nitro bedeuten, mit der Massgabe, dass $R_2$, $R_3$, $R_4$ und $R_5$ nicht gleichzeitig die Bedeutung von Wasserstoff haben.

Von besonderem Interesse sind diejenigen der vorerwähnten neuen Verbindungen der Formel I, worin $R_1$ $C_1$—$C_{10}$-Alkyl, Phenyl oder durch Fluor, Chlor, Brom oder $C_1$—$C_5$-Alkoxy substituiertes $C_1$—$C_{10}$-Alkyl; und $R_2$, $R_3$, $R_4$ und $R_5$ unabhängig voneinander Wasserstoff, Fluor, Chlor oder Brom, $C_1$—$C_5$-Alkyl, $C_1$—$C_5$-Alkoxy, Trifluormethyl, Amino oder Nitro bedeuten, mit der Massgabe, dass $R_2$, $R_3$, $R_4$ und $R_5$ nicht gleichzeitig die Bedeutung von Wasserstoff haben, sowie diejenigen, worin $R_1$ $C_1$—$C_5$-Alkyl, Phenyl oder durch 1 bis 7 Fluor- oder Chloratome substituiertes $C_1$—$C_3$-Alkyl; $R_2$ Fluor, Chlor, Brom, $C_1$—$C_5$-Alkyl, $C_1$—$C_5$-Alkoxy oder Trifluormethyl und $R_3$, $R_4$ und $R_5$ unabhängig voneinander Wasserstoff, Fluor, Chlor, Brom, $C_1$—$C_3$-Alkyl, Methoxy oder Trifluormethyl bedeuten.

Speziell bevorzugt aufgrund ihrer hohen pestiziden, vor allem aphiziden, Wirkung werden solche neuen Verbindungen der Formel I, worin $R_1$ Methyl und/oder $R_2$ Chlor, $R_3$ Wasserstoff oder Chlor und $R_4$ und $R_5$ Wasserstoff bedeuten.

Aus der japanischen Patentpublikation Sho 48—24735 sind bereits Benzisothiazol-S,S-dioxide mit fungizider und bakterizider Wirkung bekannt, wobei dort unter anderem in allgemeiner Form auch auf entsprechende 3-Acylamino-derivate mit substituiertem anellierten Benzolring Bezug genommen wird. Weiterhin sind aus der europäischen Patentanmeldung Nr. 0033984 substituierte 3-Amino-benzisothiazol-S,S-dioxide mit aphizider Wirkung bekannt.

Es Wurde nun erfindungsgemäss überraschenderweise gefunden, dass die 3-Acylamino-benzisothiazol-S,S-dioxide der Formel I ausgezeichnete Wirkung als Insektizide, speziell als Aphizide, aufweisen. Besonders hohe biologische Wirksamkeit besitzen die vorstehend definierten erfindungsgemässen neuen Verbindungen der Formel I, bei denen mindestens einer der Reste $R_2$, $R_3$, $R_4$ und $R_5$ eine von Wasserstoff abweichende Bedeutung innerhalb der gegebenen Definition hat.

Die oben genannten Verbindungen der Formel I können analog bekannten Verfahren hergestellt werden (vgl. J. March, "Advanced Organic Chemistry"; McGraw Hill, New York, 1977, S. 383—385), indem man ein 3-Amino-benzisothiazol-S,S-dioxid der Formel II

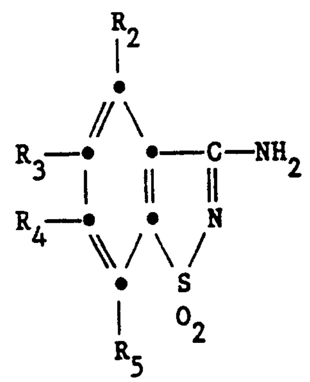

(II)

mit einem Säurehalogenid der Formel III

$$X—COR_1 \quad (III)$$

oder mit einem Säureanhydrid der Formel IV

$$R_1OC—O—COR_1 \quad (IV)$$

gegebenenfalls in Gegenwart einer Base umsetzt, wobei in den Formeln II bis IV die Reste $R_1$ bis $R_5$ die unter Formel I angegebenen Bedeutungen haben und X Halogen, vorzugsweise Chlor, bedeutet.

Als geeignete Basen kommen insbesondere tertiäre Amine, wie Trialkylamine, Dialkylaniline und p-Dialkylaminopyridine, in Betracht. Die Verfahren werden bei normalem Druck, bei einer Temperatur von −25° bis 150°C, vorzugsweise 50° bis 100°C, und gegebenenfalls in einem Lösungs- oder Verdünnungsmittel durchgeführt.

Als Lösungs- oder Verdünnungsmittel eignen sich z.B. Aether und ätherartige Verbindungen wie Diäthyläther, Diisopropyläther, Dioxan und Tetrahydrofuran; aromatische Kohlenwasserstoffe wie Benzol, Toluol und Xylole; Ketone wie Aceton, Methyläthylketon und Cyclohexanon; Nitrile wie Acetonitril; Ester wie Aethylacetat und Butylacetat; sowie Dimethylformamid, Dimethylsulfoxid, Methylcyanid und halogenierte Kohlenwasserstoffe.

Die Verbindungen der Formel II können, entsprechend den Verbindungen der Formel Ia auch in ihrer tautomeren Form der folgenden Formel IIa

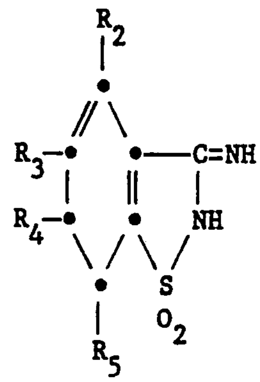

(IIa)

vorliegen.

Die Ausgangsstoffe der Formeln II, III und IV sind bekannt oder können nach bekannten Verfahren hergestellt werden. So wird die Herstellung von 3-Amino-benzisothiazol-S,S-dioxiden der Formel II in der europäischen Patentanmeldung Nr. 0033984 beschrieben.

Verbindungen der Formel I sind zur Bekämpfung von Insekten und Vertretern der Ordnung Akarina an Tieren und Pflanzen geeignet. Ferner haben diese Verbindungen zum Teil auch fungizide und pflanzenregulatorische Eigenschaften.

Insbesondere eignen sich die Verbindungen der Formel I zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Milben und Zecken aus der Ordnung Akarina.

Vor allem eignen sich Verbindungen der Formel I zur Bekämpfung von pflanzenschädigenden Insekten, speziell pflanzenschädigenden, Insekten in Zier- und Nutzpflanzungen, insbesondere in Baumwollkulturen, Gemüsekulturen, Reiskulturen und Obstkulturen. In diesem Zusammenhang ist hervorzuheben, dass die genannten Verbindungen sich sowohl durch eine stark ausgeprägte systemische als auch Kontakt-Wirkung gegen saugende Insekten, vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Wirkstoffe der Formel I zeigen auch eine günstige Wirkung gegen fressende und beissende Insekten sowie gegen Fliegen, wie z.B. Musca domestica und Mückenlarven.

Die Verbindungen der Formel I können ferner zur Bekämpfung von Ektoparasiten, wie Lucilia sericata, an Haus- und Nutztieren eingesetzt werden, z.B. durch Tier-, Stall- und Wiedebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht: organische Phosphorverbindungen, Nitrophenole und Derivate, Formamidine, Harnstoffe, Carbamate, chlorierte Kohlenwasserstoffe, Pyrethroide und Bacillus thuringiensis-Präparate.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphortrithioate.

Die gute insektizide Wirkung der erfindungsgemäss vorgeschlagenen Verbindungen der Formel I entspricht einer Abtötungsrate (Mortalität) von mindestens 50—60% der erwähnten Schadinsekten.

Die Verbindungen der Formel I werden in unveränderter Form oder vorzugsweise zusammen mit den in der Formulierungstechnik üblichen Hilfsmittel eingesetzt und werden daher z.B. zu Emulsionen direkt versprühbaren oder verdünnbaren Lösungen, verdünnten Emulsionen, Spritzpulvern, löslichen Pulvern, Stäubemittel, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungemittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon,

Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anroganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$—$C_{22}$), wie z.B. die Na- oder K-Salze oder Oel- oder Stearinsäure, oder von natürlichen Fettsäuregemischen, die z.B. aus Kokosnuss- oder Talgöl gewonnen werden können. Ferner sind als Tenside auch die Fettsäuremethyl-taurinsalze sowie modifizierte und nicht modifizierte Phospholipide zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8—22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Aethylenglykoleinheiten.

Als Beispiele nichtionischer Tenside seien Nonylphenolpolyäthoxyäthanole, Ricinusölpolyglykoläther, Polypropylenpolyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi-(2-chloräthyl)-äthylammoniumbromid.

Die in der Formulierungstechik gebräuchlichen Tenside sind u.a. in folgender Publikation beschrieben:

McCutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für feste Wirkstoffe der Formel I (% = Gewichtsprozent)

1. *Spritzpulver*

| | a) | b) | c) |
|---|---|---|---|
| Wirkstoff | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyäthylenglykol-äther (7—8 Mol AeO) | — | 2% | — |
| Hochdisperse Kieselsäure | 5% | 10% | 10% |
| Kaolin | 67% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. *Stäubemittel*

| | a) | b) |
|---|---|---|
| Wirkstoff | 5% | 8% |
| Talkum | 95% | — |
| Kaolin | — | 92% |

Man erhält anwendungsfertige Stäubemittel, indem der Wirkstoff mit dem Träger vermischt und auf einer geeigneten Mühle vermahlen wird.

3. *Extruder Granulat*

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

4. *Umhüllungs-Granulat*

| | |
|---|---|
| Wirkstoff | 3% |
| Polyäthylenglykol (M.G. 200) | 3% |
| Kaolin | 94% |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

5. *Suspensions-Konzentrat*

| | |
|---|---|
| Wirkstoff | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

Herstellung von 3-Chloracetylamino-benzisothiazol-S,S-dioxid

12,75 g 3-Amino-benzisothiazol-S,S-dioxyd und 50 g Chloressigsäureanhydrid werden 10 Stunden bei 100°C gerührt. Das Reaktionsgemisch wird dann in 1 Liter Toluol gegeben. Der Niederschlag wird abgenutscht und auf der Nutsche mit Toluol und Aether gewaschen.

Nach dem Trocknen im Vakuum erhält man die Titelverbindung (Verbindung Nr. 1) der Formel

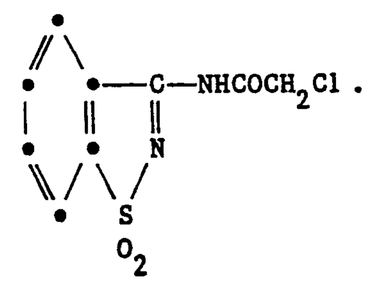

mit einem Schmelzpunkt von 183—186°C.

Beispiel 2

Herstellung von 3-Acetylamino-4-chlor-benzisothiazol-S,S-dioxid

10,2 g 3-Amino-4-chlorbenzisothiazol-S,S-dioxid und 70 ml Acetanhydrid werden während 48 Stunden bei 100°C gerührt. Das Reaktionsgemisch wird abgekühlt, die ausgeschiedenen Kristalle abgesaugt, mit Wasser gewaschen und bei 80°C getrocknet.

Man erhält die Titelverbindung als hellgelbe Kristalle vom Schmelzpunkt 210—213°C (Verbindung Nr. 5).

Auf analoge Weise wie oben angegeben werden auch folgende Verbindungen der Formel I hergestellt:

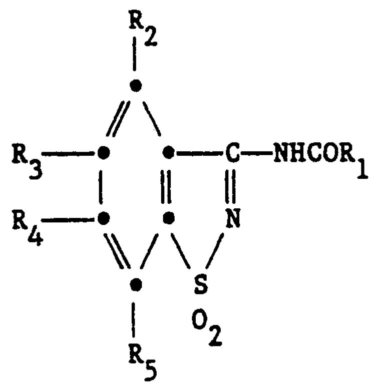

(I) ,

| Nr. | $R_1$ | $R_2$ | $R_3$ | $R_4$ | $R_5$ | Physikalische Daten |
|---|---|---|---|---|---|---|
| 2 | $-C_2H_5$ | H | H | H | H | Smp.: 233-236°C |
| 3 | $-C_3H_7$(n) | H | H | H | H | Smp.: 211-213°C |
| 4 | 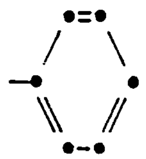 | H | H | H | H | Smp.: 230-235°C |
| 5 | $-CH_3$ | Cl | H | H | H | Smp.: 210-213°C |
| 6 | $-C_2H_5$ | Cl | H | H | H | Smp.: 176-178°C |
| 7 | 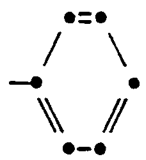 | Cl | H | H | H | Smp.: > 250°C |
| 8 | $-CH_3$ | H | H | H | H | Smp.: 264-265°C |
| 9 | $-CH_2Cl$ | Cl | H | H | H | Smp.: 181-185°C |
| 10 | $-CF_3$ | Cl | H | H | H | Smp.: 201-202°C |
| 11 | $-C_3H_7$(i) | Cl | H | H | H | Smp.: 187°C |
| 12 | $-C_5H_{11}$(n) | H | H | H | H | Smp.: 219-221°C |
| 13 | $-C_5H_{11}$(n) | Cl | H | H | H | Smp.: 120-123°C |
| 14 | $-CF_2CF_2CF_3$ | H | H | H | H | Smp.: 189-192°C |
| 15 | $-CF_2CF_2CF_3$ | Cl | H | H | H | Smp.: 245-246°C |
| 16 | $-CF_2-CF_3$ | H | H | H | H | Smp.: 234-236°C |
| 17 | $-CF_2-CF_3$ | Cl | H | H | H | Smp.: 241-242°C |
| 18 | $-CCl_3$ | H | H | H | H | Smp.: 210-212°C |
| 19 | $-CCl_3$ | Cl | H | H | H | Smp.: 244-247°C |
| 20 | $-CF_3$ | H | H | H | H | Smp.: 236-238°C |
| 21 | $-C_3H_7$(i) | H | H | H | H | Smp.: 267-270°C |
| 22 | $-CH_3$ | Cl | Br | H | H | Smp.: 225-230°C |
| 23 | $-C_2H_5$ | Cl | Br | H | H | Smp.: 237-240°C |
| 24 | $-CH_3$ | H | $CH_3$ | H | H | Smp.: 283-286°C |
| 25 | $-C_3H_7$(n) | Cl | H | H | H | Smp.: 186-188°C |
| 26 | $-C_2H_5$ | Cl | H | H | Cl | Smp.: 240-143°C |
| 27 | $-CH_3$ | Cl | H | H | Cl | Smp.: 235-238°C |
| 28 | $-CH_3$ | H | H | Cl | H | Smp.: > 260°C |
| 29 | $-CH_3$ | H | H | H | Cl | Smp.: 245-248°C |
| 30 | $-CH_3$ | $CH_3$ | H | H | H | Smp.: 242-245°C |
| 31 | $-CH_3$ | F | H | H | H | Smp.: 209-212°C |

Die folgenden Verbindungen der Formel I sind ebenfalls wie oben beschrieben herstellbar:

| Nr. | $R_1$ | $R_2$ | $R_3$ | $R_4$ | $R_5$ |
|---|---|---|---|---|---|
| 32 | $-CH_3$ | H | Cl | H | $-OCH_3$ |
| 33 | $-CH_3$ | Cl | H | H | $-NO_2$ |
| 34 | $-CH_3$ | $-CH_3$ | H | $-CH_3$ | $-CH_3$ |

### Beispiel 3

Insektizide Kontaktwirkung: Aphis craccivora

In Töpfen angezogene Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 0.75, 12.5, 50, 100, 200 und 400 ppm der zu prüfenden Verbindung bis zur Tropfnässe besprüht. Man verwendet pro Test-Verbindung und pro Konzentration zwei Pflanzen, und eine Auswertung der erzielten Abtötungsrate erfolgt nach weiteren 24 Stunden.

### Beispiel 4

Insektizide systemische Wirkung: Aphis craccivora

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25%igen Spritzpulver) jeweils in einer Konzentration von 0.75, 3, 12.5 oder 50 ppm direkt auf die Erde in den Töpfen.

Nach 24 Stunden werden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgt 48 und 72 Stunden nach Versuchsbeginn. Pro Konzentrationsdosis Testsubstanz werden zwei Pflanzen, je eine in einem separaten Topf, verwendet. Der Versuch wird bei 25°C und 70% relativer Luftfeuchtigkeit durchgeführt.

### Beispiel 5

Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 50, 100 oder 200 ppm der zu prüfenden Verbindung bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20—22°C und 60% relativer Luftfeuchtigkeit durchgeführt.

### Beispiel 6

Insektizide zystemische Wirkung: Myzus persicae

Bewurzelte Kohlpflanzen im 4- bis 5-Blatt-Stadium werden in Töpfe, welche 60 ccm Erde enthalten, verpflanzt. Anschliessend werden 50 ml einer wässrigen Formulierung der zu prüfenden Verbindung I (erhalten aus einem 25%igen Spritzpulver) jeweils in einer Konzentration von 0.75, 3 bzw. 12.75 ppm direkt auf die Erde aufgegossen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen Kontakt- oder Gaswirkung der Testsubstanz zu schützen.

Die Auswertung der erzielten %-Abtötung erfolgt 48 Stunden nach Versuchsbeginn. Pro Konzentrationsdosis Testsubstanz werden zwei Pflanzen, je eine in separaten Töpfen, verwendet. Der Versuch wird bei ca. 25°C und 60% relativer Luftfeuchtigkeit durchgeführt.

### Beispiel 7

Insektizide Blattpenetrations-Wirkung: Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die

Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 100 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60% relativer Luftfeuchtigkeit durchgeführt. Die Auswertung auf %-Mortalität erfolgt 48 Stunden nach Wirkstoffapplikation.

Biologische Versuchsergebnisse

In der folgenden Tabelle sind Versuchsergebnisse auf der Basis der vorstehenden Beispiele 3 bis 7 aufgeführt, und zwar mit folgendem Bewertungsindex in Bezug auf die prozentuale Abtötung der Schädlinge ("—" bedeutet "nicht geprüft"):

A: 80—100% Abtötung bei 0,75 ppm Wirkstoffkonzentration
B: 80—100% Abtötung bei 3,0 ppm Wirkstoffkonzentration
C: 80—100% Abtötung bei 12,5 ppm Wirkstoffkonzentration
D: 80—100% Abtötung bei 50 ppm Wirkstoffkonzentration
E: 80—100% Abtötung bei 100 ppm Wirkstoffkonzentration
F: 80—100% Abtötung bei 200 ppm Wirkstoffkonzentration
G: 80—100% Abtötung bei 400 ppm Wirkstoffkonzentration

| Verb. Nr. | Kontaktwirkung | | Systemische Wirkung | | Blattpenetrations-Wirkung |
|---|---|---|---|---|---|
| | Myzus | Aphis | Myzus | Aphis | Aphis |
| 1 | D | E | B | C | E |
| 2 | - | E | C | C | - |
| 3 | F | F | - | C | - |
| 4 | F | F | - | C | - |
| 5 | E | C | B | B | E |
| 6 | E | D | B | B | - |
| 7 | F | F | B | C | - |
| 8 | - | F | B | C | - |
| 9 | F | D | B | B | E |
| 10 | - | G | C | C | E |
| 11 | E | F | A | A | E |
| 13 | D | D | B | A | E |
| 15 | E | G | B | B | E |
| 17 | D | G | A | C | E |
| 19 | D | G | A | A | E |
| 25 | D | A | B | D | E |

## Patentansprüche

1. Verwendung einer Verbindung der Formel I

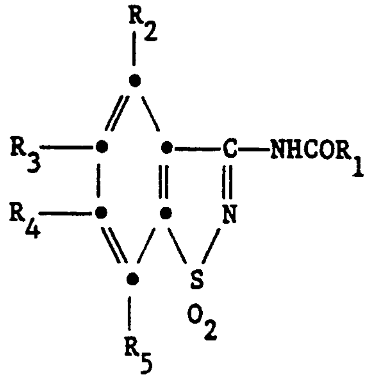

(I) ,

worin $R_1$ $C_1$—$C_{10}$-Alkyl, Phenyl oder durch Halogen oder $C_1$—$C_5$-Alkoxy substituiertes $C_1$—$C_{10}$-Alkyl; und $R_2$, $R_3$, $R_4$ und $R_5$ unabhängig voneinander Wasserstoff, Halogen, $C_1$—$C_5$-Alkyl, $C_1$—$C_5$-Alkoxy, Trifluormethyl, Amino oder Nitro bedeuten, zur Bekämpfung von Insekten und Vertretern der Ordnung Akarina.

2. Verwendung gemäss Anspruch 1, dadurch gekennzeichnet, dass in der Verbindung der Formel I $R_1$ $C_1$—$C_{10}$-Alkyl, Phenyl oder durch Fluor, Chlor, Brom oder $C_1$—$C_5$-Alkoxy substituiertes $C_1$—$C_{10}$-Alkyl; und $R_2$, $R_3$, $R_4$ und $R_5$ unabhängig voneinander Wasserstoff, Fluor, Chlor, Brom, $C_1$—$C_5$-Alkyl, $C_1$—$C_5$-Alkoxy, Trifluormethyl, Amino oder Nitro bedeuten.

3. Verwendung gemäss Anspruch 2, dadurch gekennzeichnet, dass in der Verbindung der Formel I $R_1$ $C_1$—$C_5$-Alkyl, Phenyl oder durch 1 bis 7 Fluor- oder Chloratome substituiertes $C_1$—$C_3$-Alkyl; $R_2$ Fluor, Chlor, Brom, $C_1$—$C_5$-Alkyl, $C_1$—$C_5$-Alkoxy oder Trifluormethyl und $R_3$, $R_4$ und $R_5$ unabhängig voneinander Wasserstoff, Fluor, Chlor, Brom, $C_1$—$C_3$-Alkyl, Methoxy oder Trifluormethyl bedeuten.

4. Verwendung gemäss Anspruch 1 bis 3, dadurch gekennzeichnet, dass in der Verbindung der Formel I $R_1$ Methyl bedeutet.

5. Verwendung gemäss Anspruch 1 bis 4, dadurch gekennzeichnet, dass in der Verbindung der Formel I $R_2$ Chlor, $R_3$ Wasserstoff oder Chlor, und $R_4$ und $R_5$ Wasserstoff bedeuten.

6. Verwendung gemäss Anspruch 2 der Verbindung der Formel

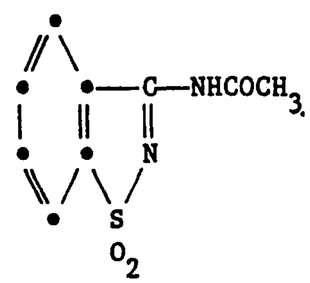

7. Verwendung gemäss Anspruch 2 der Verbindung der Formel

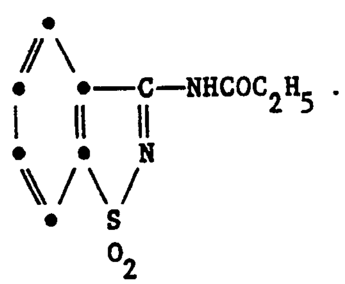

8. Verwendung gemäss Anspruch 2 der Verbindung der Formel

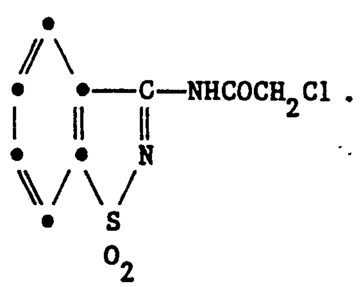

9. Verwendung gemäss Anspruch 5 der Verbindung der Formel

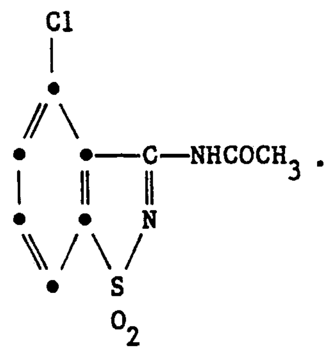

10. Verwendung gemäss Anspruch 5 der Verbindung der Formel

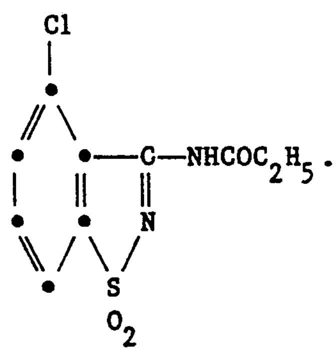

11. Verwendung gemäss Anspruch 5 der Verbindung der Formel

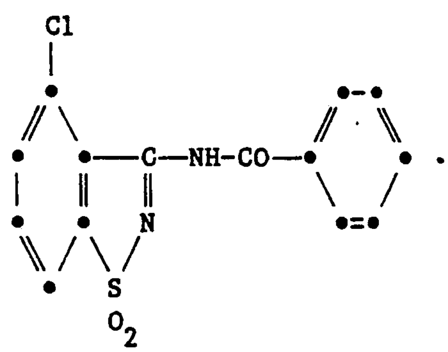

12. Verwendung gemäss Anspruch 5 der Verbindung der Formel

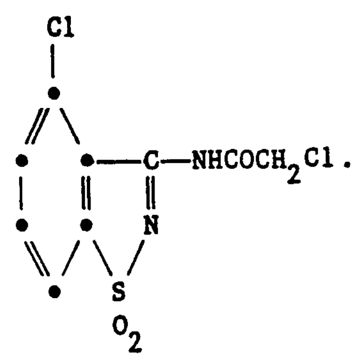

13. Verwendung gemäss Anspruch 5 der Verbindung der Formel

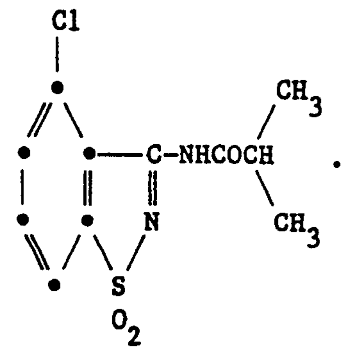

14. Verwendung gemäss Anspruch 5 der Verbindung der Formel

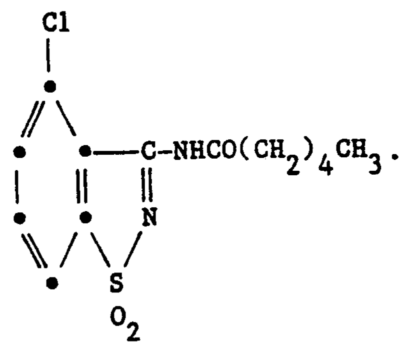

15. Verwendung gemäss Anspruch 5 der Verbindung der Formel

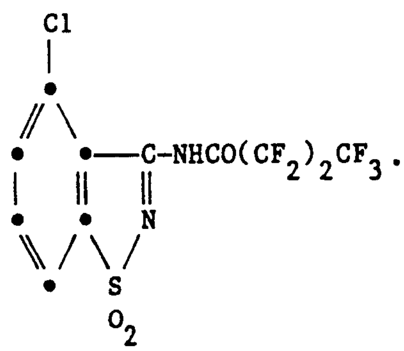

16. Verwendung gemäss Anspruch 5 der Verbindung der Formel

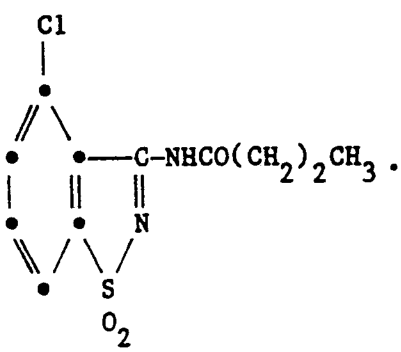

17. Verwendung gemäss Anspruch 5 der Verbindung der Formel

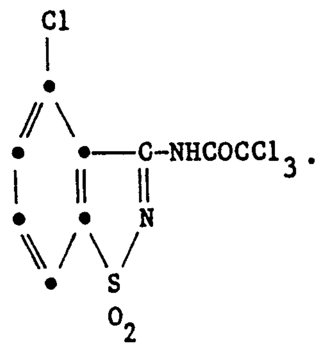

18. Verwendung gemäss Anspruch 1 bis 17 zur Bekämpfung pflanzenschädigender Insekten.

19. Verwendung gemäss Anspruch 18 zur Bekämpfung von Aphiden.

20. Verfahren zur Bekämpfung von Insekten und Vertretern der Ordnung Akarina, dadurch gekennzeichnet, dass man die Schädlinge oder ihren Aufenthaltsort mit einer pestizid wirksamen Menge einer Verbindung der Formel I, wie in den Ansprüchen 1 bis 17 definiert, oder einem Mittel enthaltend neben Zusatz- und Trägerstoffen eine pestizid wirksame Menge dieser Verbindung, in Kontakt bringt oder behandelt.

21. Verbindung der Formel I

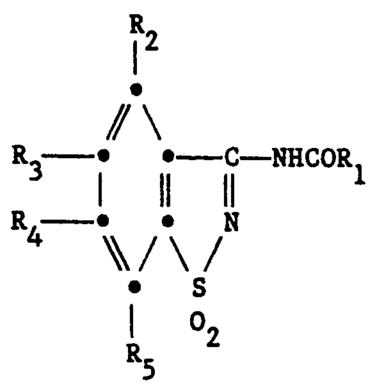

(I)

worin $R_1$ $C_1$—$C_{10}$-Alkyl, Phenyl oder durch Halogen oder $C_1$—$C_5$-Alkoxy substituiertes $C_1$—$C_{10}$-Alkyl; und $R_2$, $R_3$, $R_4$ und $R_5$ unabhängig voneinander Wasserstoff, Halogen, $C_1$—$C_5$-Alkyl, $C_1$—$C_5$-Alkoxy, Trifluormethyl, Amino oder Nitro bedeuten, mit der Massgabe, dass $R_2$, $R_3$, $R_4$ und $R_5$ nicht gleichzeitig die Bedeutung von Wasserstoff haben.

22. Verbindung gemäss Anspruch 21 der Formel I, worin $R_1$ $C_1$—$C_{10}$-Alkyl, Phenyl oder durch Fluor, Chlor, Brom oder $C_1$—$C_5$-Alkoxy substituiertes $C_1$—$C_{10}$-Alkyl; und $R_2$, $R_3$, $R_4$ und $R_5$ unabhängig voneinander Wasserstoff, Fluor, Chlor oder Brom, $C_1$—$C_5$-Alkyl, $C_1$—$C_5$-Alkoxy, Trifluormethyl, Amino oder Nitro bedeuten, mit der Massgabe, dass $R_2$, $R_3$, $R_4$ und $R_5$ nicht gleichzeitig die Bedeutung von Wasserstoff haben.

23. Verbindung gemäss Anspruch 22 der Formel I, worin $R_1$ $C_1$—$C_5$-Alkyl, Phenyl oder durch 1 bis 7 Fluor- oder Chloratome substituiertes $C_1$—$C_3$-Alkyl; $R_2$ Fluor, Chlor, Brom, $C_1$—$C_5$-Alkyl, $C_1$—$C_5$-Alkoxy oder Trifluormethyl und $R_3$, $R_4$ und $R_5$ unabhängig voneinander Wasserstoff, Fluor, Chlor, Brom, $C_1$—$C_3$-Alkyl, Methoxy oder Trifluormethyl bedeuten.

24. Verbindung gemäss Anspruch 21 bis 23 der Formel I, worin $R_1$ Methyl bedeutet.

25. Verbindung gemäss Anspruch 21 bis 24 der Formel I, worin $R_2$ Chlor, $R_3$ Wasserstoff oder Chlor, und $R_4$ und $R_5$ Wasserstoff bedeuten.

26. Verbindung gemäss Anspruch 25 der Formel

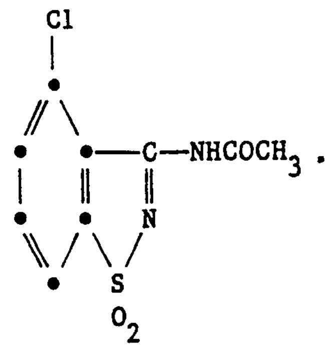

27. Verbindung gemäss Anspruch 25 der Formel

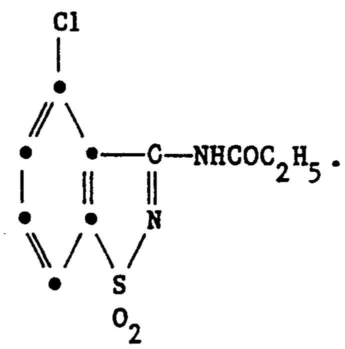

28. Verbindung gemäss Anspruch 25 der Formel

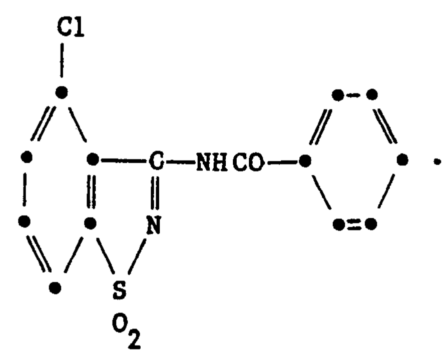

29. Verbindung gemäss Anspruch 25 der Formel

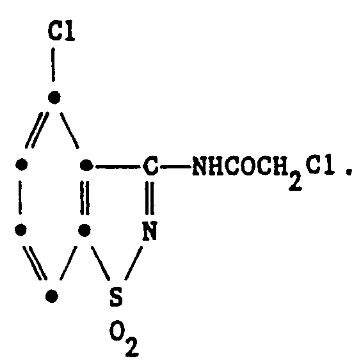

30. Verbindung gemäss Anspruch 25 der Formel

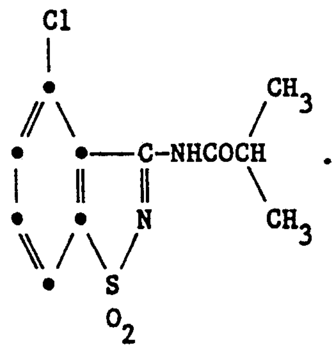

31. Verbindung gemäss Anspruch 25 der Formel

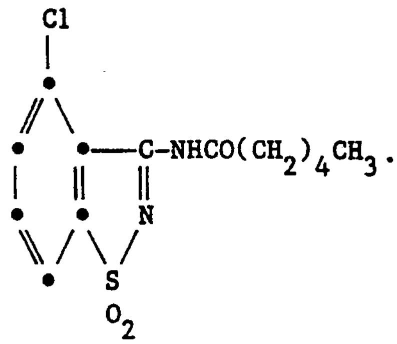

32. Verbindung gemäss Anspruch 25 der Formel

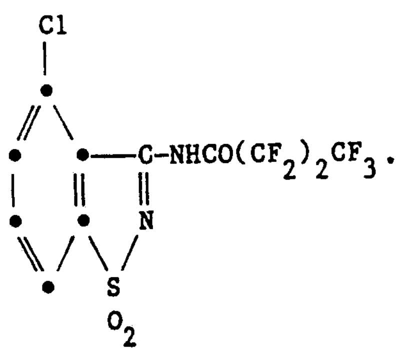

33. Verbindung gemäss Anspruch 25 der Formel

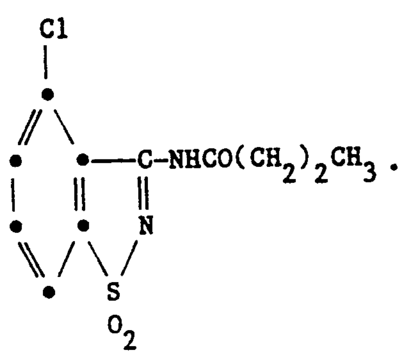

34. Verbindung gemäss Anspruch 25 der Formel

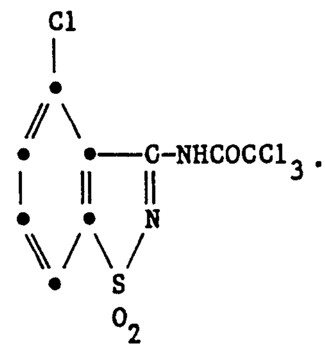

35. Verfahren zur Herstellung einer Verbindung der Formel I gemäss den Ansprüchen 21 bis 34, dadurch gekennzeichnet, dass man eine Verbindung der Formel II

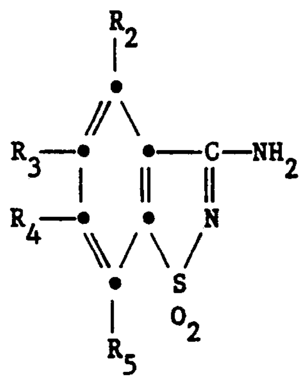

(II)

mit einer Verbindung der Formel III

$$X{-}COR_1 \quad (III)$$

oder mit einer Verbindung der Formel IV

$$R_1OC{-}O{-}COR_1 \quad (IV)$$

umsetzt, wobei in den Formeln II bis IV die Reste $R_1$ bis $R_5$ die in Anspruch 21 unter Formel I angegebenen Bedeutungen haben und X Halogen, vorzugsweise Chlor, bedeutet.

36. Schädlingsbekämpfungsmittel, dadurch gekennzeichnet, dass es neben geeigneten Träger- und/ oder andern Zuschlagstoffen als aktive Komponente eine Verbindung der Formel I gemäss Anspruch 21 bis 34 enthält.

37. Verwendung einer Verbindung der Formel I gemäss Anspruch 21 zur Bekämpfung von Pflanzen und Tieren befallenden Schädlingen, insbesondere von Insekten, Vertretern der Ordnung Akarina, Pilzen und Bakterien.

**Revendications**

1. Utilisation d'un composé de formule I

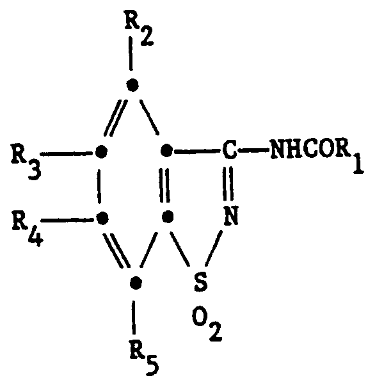

(I) ,

dans laquelle $R_1$ représente un groupe alkyle en $C_1—C_{10}$, un groupe phényle ou un groupe alkyle en $C_1—C_{10}$ substitué par des halogènes ou des groupes alcoxy en $C_1—C_5$; et $R_2$, $R_3$, $R_4$ et $R_5$ représentent chacun, indépendamment les uns des autres, l'hydrogène, un halogène, un groupe alkyle en $C_1—C_5$, alcoxy en $C_1—C_5$, trifluorométhyle, amino ou nitro, pour la lutte contre les insectes et les représentants de l'ordre des acariens.

2. Utilisation selon la revendication 1, caractérisée en ce que, dans le composé de formule I, $R_1$ représente un groupe alkyle en $C_1—C_{10}$, phényle ou un groupe alkyle en $C_1—C_{10}$ substitué par le fluor, le chlore, le brome ou des groupes alcoxy en $C_1—C_5$; et $R_2$, $R_3$, $R_4$ et $R_5$ représentent chacun, indépendamment les uns des autres, l'hydrogène, le fluor, le chlore, le brome, un groupe alkyle en $C_1—C_5$, alcoxy en $C_1—C_5$, trifluorométhyle, amino ou nitro.

3. Utilisation selon la revendication 2, caractérisée en ce que, dans le composé de formule I, $R_1$ représente un groupe alkyle en $C_1—C_5$, phényle ou un groupe alkyle en $C_1—C_3$ substitué par un à sept atomes de fluor ou de chlore; $R_2$ représente le fluor, le chlore, le brome, un groupe alkyle en $C_1—C_5$, alcoxy en $C_1—C_5$ ou trifluorométhyle et $R_3$, $R_4$ et $R_5$ représentent chacun, indépendamment les uns des autres, l'hydrogène, le fluor, le chlore, le brome, un groupe alkyle en $C_1—C_3$, méthoxy ou trifluorométhyle.

4. Utilisation selon les revendications 1 à 3, caractérisée en ce que, dans le composé de formule I, $R_1$ représente un groupe méthyle.

5. Utilisation selon les revendications 1 à 4, caractérisée en ce que, dans le composé de formule I, $R_2$ représente le chlore, $R_3$ l'hydrogène ou le chlore et $R_4$ et $R_5$ l'hydrogène.

6. Utilisation selon la revendication 2 du composé de formule:

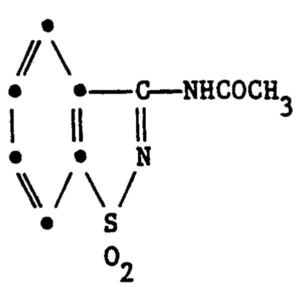

.

7. Utilisation selon la revendication 2 du composé de formule:

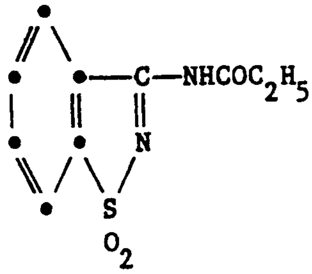

.

8. Utilisation selon la revendication 2 du composé de formule:

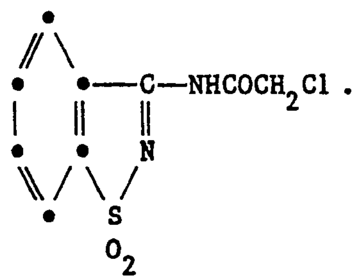

9. Utilisation selon la revendication 5 du composé de formule:

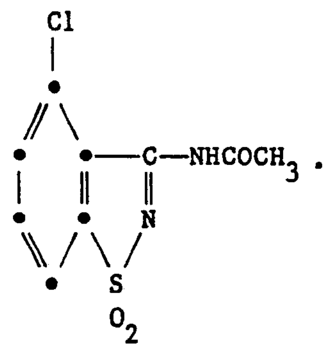

10. Utilisation selon la revendication 5 du composé de formule:

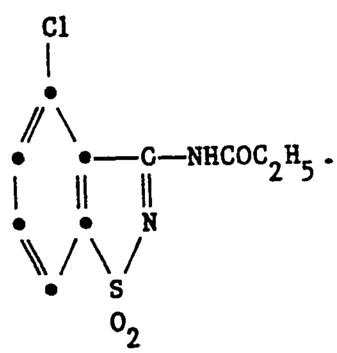

11. Utilisation selon la revendication 5 du composé de formule:

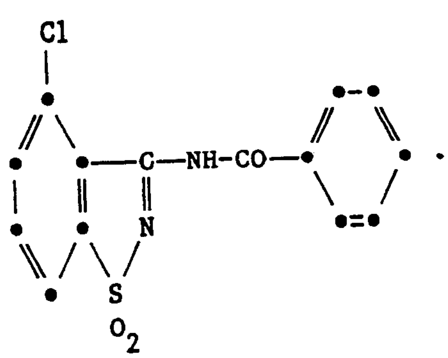

12. Utilisation selon la revendication 5 du composé de formule:

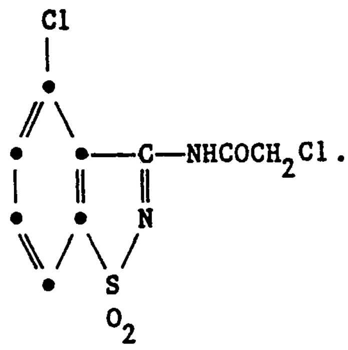

13. Utilisation selon la revendication 5 du composé de formule:

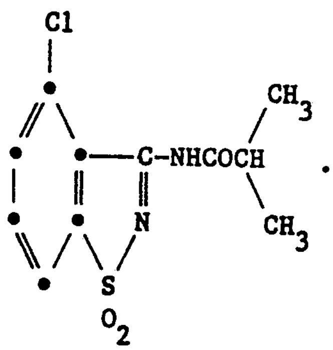

14. Utilisation selon la revendication 5 du composé de formule:

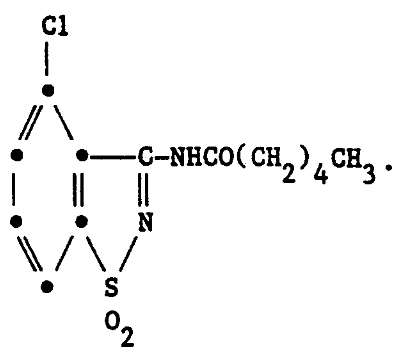

15. Utilisation selon la revendication 5 du composé de formule:

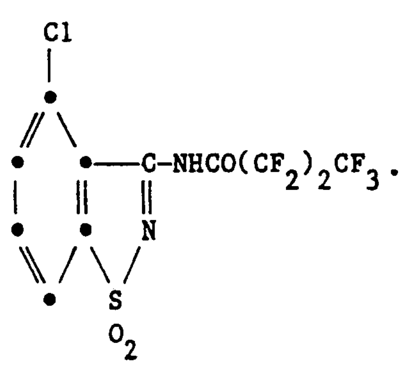

16. Utilisation selon la revendication 5 du composé de formule:

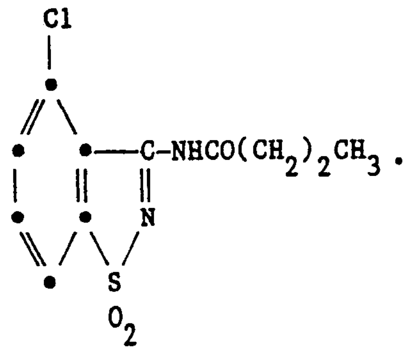

17. Utilisation selon la revendication 5 du composé de formule:

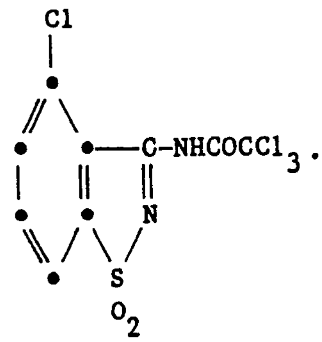

18. Utilisation selon les revendications 1 à 17 pour la lutte contre les insectes nuisibles pour les végétaux.

19. Utilisation selon la revendication 18 pour la lutte contre les aphidés.

20. Procédé pour combattre les insectes et les représentants de l'ordre des acariens, caractérisé en ce que l'on met en contact ou on traite les parasites ou leur habitat par une quantité pesticide efficace d'un composé de formule I tel que défini dans les revendications 1 à 17 ou d'un produit contenant, avec des additifs et des véhicules, une quantité pesticide efficace d'un tel composé.

21. Composé de formule I

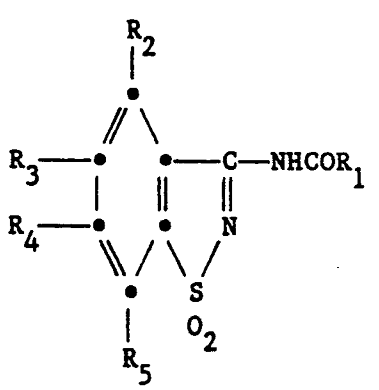

(I)

dans laquelle $R_1$ représente un groupe alkyle en $C_1$—$C_{10}$, phényle ou un groupe alkyle en $C_1$—$C_{10}$ substitué par des halogènes ou des groupes alcoxy en $C_1$—$C_5$; et $R_2$, $R_3$, $R_4$ et $R_5$ représentent chacun, indépendamment les uns des autres, l'hydrogène, un halogène, un groupe alkyle en $C_1$—$C_5$, alcoxy en $C_1$—$C_5$, trifluorométhyle, amino ou nitro, sous réserve que $R_2$, $R_3$, $R_4$ et $R_5$ ne peuvent représenter tous simultanément l'hydrogène.

22. Composé selon la revendication 21, de formule I dans laquelle $R_1$ représente un groupe alkyle en $C_1$—$C_{10}$, phényle ou un groupe alkyle en $C_1$—$C_{10}$ substitué par le fluor, le chlore, le brome ou des groupes alcoxy en $C_1$—$C_5$; et $R_2$, $R_3$, $R_4$ et $R_5$ représentent chacun, indépendamment les uns des autres, l'hydrogène, le fluor, le chlore ou le brome, un groupe alkyle en $C_1$—$C_5$, alcoxy en $C_1$—$C_5$, trifluorométhyle, amino ou nitro, sous réserve que $R_2$, $R_3$, $R_4$ et $R_5$ ne peuvent représenter tous simultanément l'hydrogène.

23. Composé selon la revendication 22, de formule I dans laquelle $R_1$ représente un groupe alkyle en $C_1$—$C_5$, phényle ou un groupe alkyle en $C_1$—$C_3$ substitué par 1 à 7 atomes de fluor ou de chlore; $R_2$ représente le fluor, le chlore, le brome, un groupe alkyle en $C_1$—$C_5$, alcoxy en $C_1$—$C_5$ ou trifluorométhyle et $R_3$, $R_4$ et $R_5$ représentent chacun, indépendamment les uns des autres, l'hydrogène, le fluor, le chlore, le brome, un groupe alkyle en $C_1$—$C_3$, méthoxy ou trifluorométhyle.

24. Composé selon les revendications 21 à 23, de formule I dans laquelle $R_1$ représente un groupe méthyle.

25. Composé selon les revendications 21 à 24, de formule I dans laquelle $R_2$ représente le chlore, $R_3$ l'hydrogène ou le chlore et $R_4$ et $R_5$ l'hydrogène.

26. Composé selon la revendication 25, de formule:

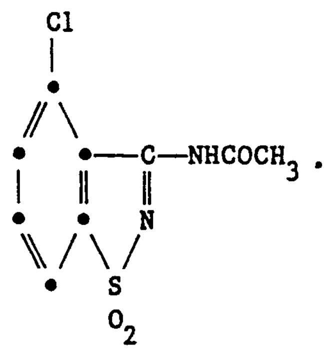

27. Composé selon la revendication 25, de formule:

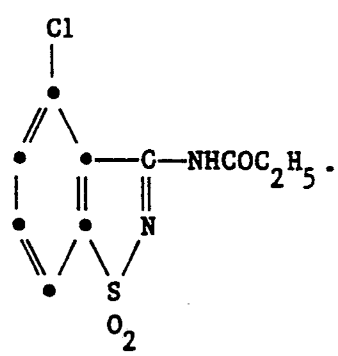

28. Composé selon la revendication 25, de formule:

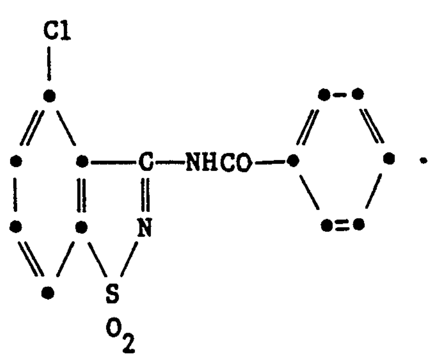

29. Composé selon la revendication 25, de formule:

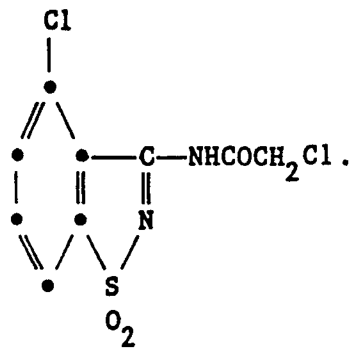

30. Composé selon la revendication 25, de formule:

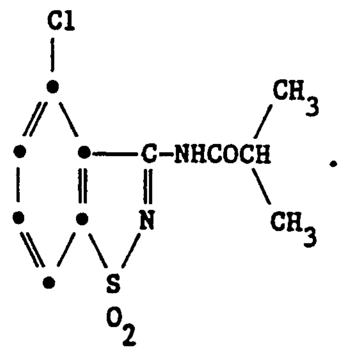

31. Composé selon la revendication 25, de formule:

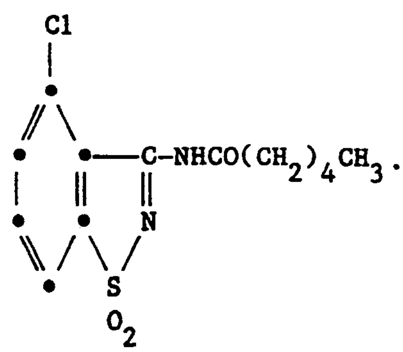

32. Composé selon la revendication 25, de formule:

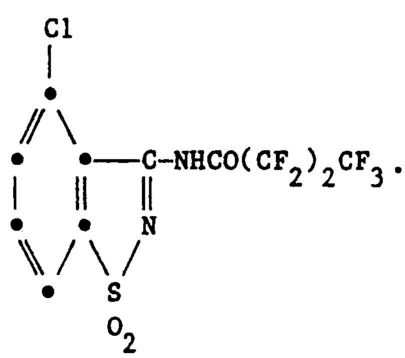

33. Composé selon la revendication 25, de formule:

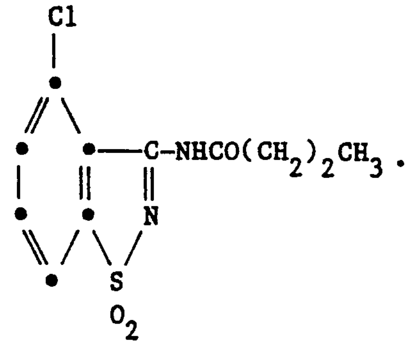

34. Composé selon la revendication 25, de formule:

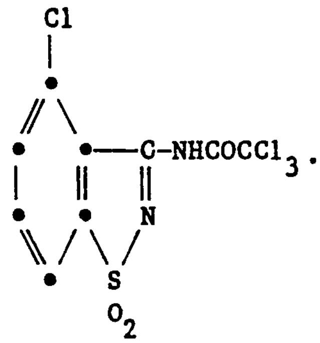

35. Procédé de préparation d'un composé de formule I selon les revendications 21 à 34, caractérisé en ce que l'on fait réagir un composé de formule II,

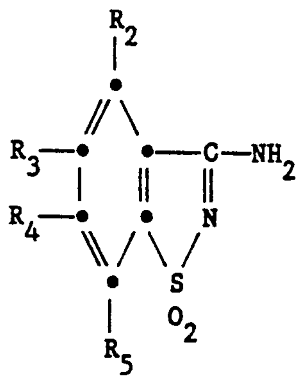

(II)

avec un composé de formule III

$$X—COR_1 \quad (III)$$

ou avec un composé de formule IV

$$R_1OC—O—COR_1 \quad (IV)$$

les symboles $R_1$ à $R_5$ ayant dans les formules II à IV les significations indiquées en référence à la formule I dans la revendication 21, et X représentant un halogène, de préférence le chlore.

36. Produit pesticide, caractérisé en ce qu'il contient en tant que composant actif, avec des véhicules et/ou d'autres additifs appropriés, un composé de formule I selon les revendications 21 à 34.

37. Utilisation d'un composé de formule I selon la revendication 21, pour la lutte contre les parasites infestant les végétaux et les animaux, en particulier les insectes, les représentants de l'ordre des acariens, les mycètes et les bactéries.

**Claims**

1. Use of a compound of the formula I

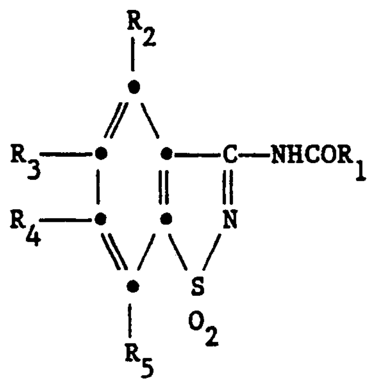

(I)

in which $R_1$ is $C_1$—$C_{10}$alkyl, phenyl, or $C_1$—$C_{10}$alkyl which is substituted by halogen or $C_1$—$C_5$alkoxy, and $R_2$, $R_3$, $R_4$ and $R_5$ are each independently of one another hydrogen, halogen, $C_1$—$C_5$alkyl, $C_1$—$C_5$alkoxy, trifluoromethyl, amino or nitro, for controlling insects and representatives of the order Acarina.

2. Use according to claim 1, wherein in the compound of the formula I $R_1$ is $C_1$—$C_{10}$alkyl, phenyl, or $C_1$—$C_{10}$alkyl which is substituted by fluorine, chlorine, bromine or $C_1$—$C_5$alkoxy, and $R_2$, $R_3$, $R_4$ and $R_5$ are each independently of one another hydrogen, fluorine, chlorine, bromine, $C_1$—$C_5$alkyl, $C_1$—$C_5$alkoxy, trifluoromethyl, amino or nitro.

3. Use according to claim 2, wherein in the compound of the formula I $R_1$ is $C_1$—$C_5$alkyl, phenyl, or $C_1$—$C_3$alkyl which is substituted by 1 to 7 fluorine or chlorine atoms, $R_2$ is fluorine, chlorine, bromine, $C_1$—$C_5$alkyl, $C_1$—$C_5$alkoxy or trifluoromethyl, and $R_3$, $R_4$ and $R_5$ are each independently of one another hydrogen, fluorine, chlorine, bromine, $C_1$—$C_3$-alkyl, methoxy or trifluoromethyl.

4. Use according to any one of claims 1 to 3, wherein in the compound of the formula I $R_1$ is methyl.

5. Use according to any one of claims 1 to 4, wherein in the compound of the formula I $R_2$ is chlorine, $R_3$ is hydrogen or chlorine, and $R_4$ and $R_5$ are hydrogen.

6. Use according to claim 2 of the compound of the formula

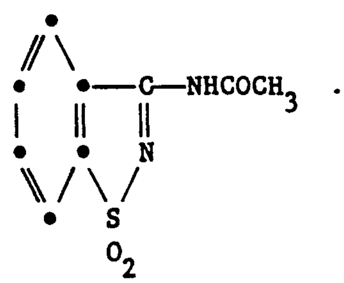

7. Use according to claim 2 of the compound of the formula

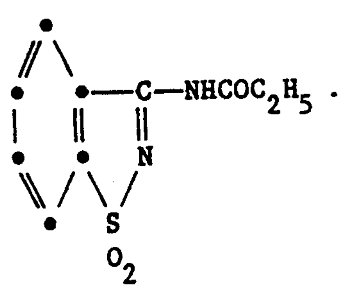

8. Use according to claim 2 of the compound of the formula

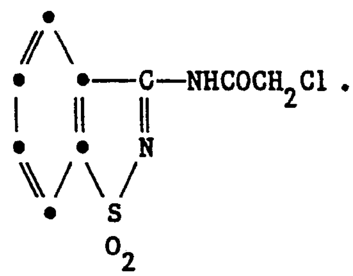

9. Use according to claim 5 of the compound of the formula

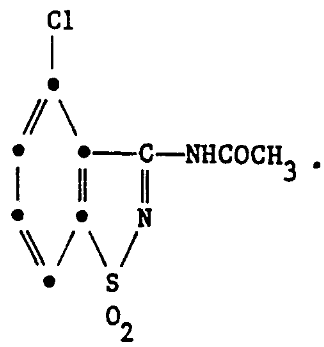

10. Use according to claim 5 of the compound of the formula

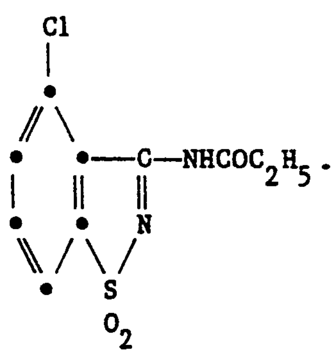

11. Use according to claim 5 of the compound of the formula

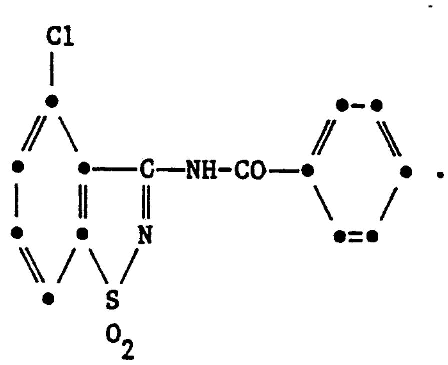

12. Use according to claim 5 of the compound of the formula

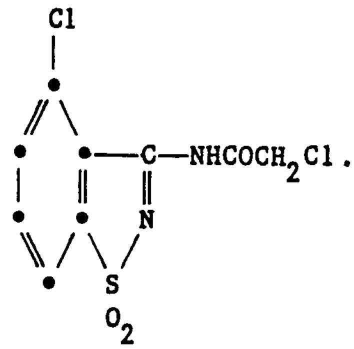

13. Use according to claim 5 of the compound of the formula

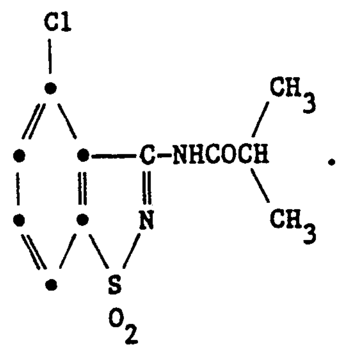

14. Use according to claim 5 of the compound of the formula

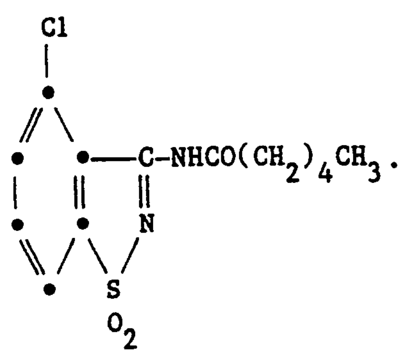

15. Use according to claim 5 of the compound of the formula

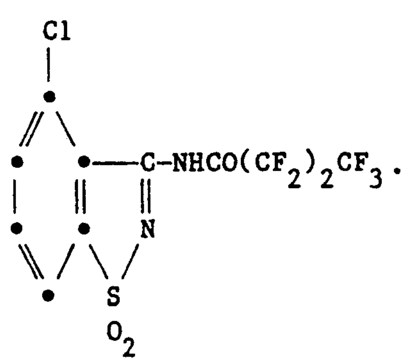

16. Use according to claim 5 of the compound of the formula

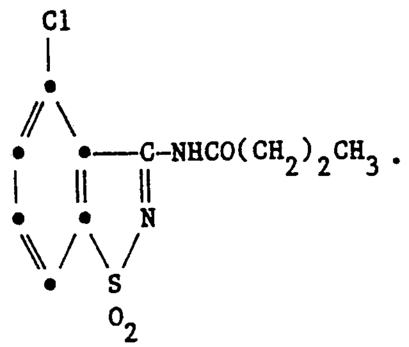

17. Use according to claim 5 of the compound of the formula

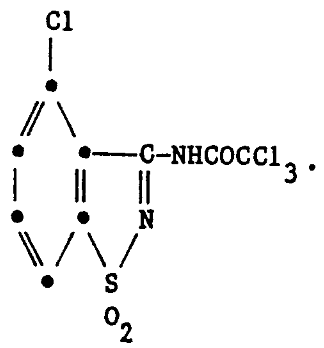

18. Use according to any one of claims 1 to 17 for controlling plant-destructive insects.

19. Use according to claim 18 for controlling aphids.

20. A method of controlling insects and representatives of the order Acarina, which comprises contacting or treating these pests or the locus thereof with a pesticidally effective amount of a compound of the formula I as defined in any one of claims 1 to 17, or with a composition containing a pesticidally effective amount of this compound, together with adjuvants and carriers.

21. A compound of the formula I

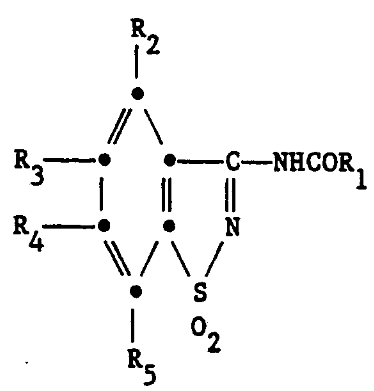

(I)

in which $R_1$ is $C_1$—$C_{10}$alkyl, phenyl, or $C_1$—$C_{10}$alkyl which is substituted by halogen or $C_1$—$C_5$alkoxy, and $R_2$, $R_3$, $R_4$ and $R_5$ are each independently of one another hydrogen, halogen, $C_1$—$C_5$alkyl, $C_1$—$C_5$alkoxy, trifluoromethyl, amino or nitro, with the proviso that $R_2$, $R_3$, $R_4$ and $R_5$ are not simultaneously hydrogen.

22. A compound according to claim 21 of the formula I, in which $R_1$ is $C_1$—$C_{10}$alkyl, phenyl, or $C_1$—$C_{10}$alkyl which is substituted by fluorine, chlorine, bromine or $C_1$—$C_5$alkoxy, and $R_2$, $R_3$, $R_4$ and $R_5$ are each independently of one another hydrogen, fluorine, chlorine or bromine, $C_1$—$C_5$alkyl, $C_1$—$C_5$alkoxy, trifluoromethyl, amino or nitro, with the proviso that $R_2$, $R_3$, $R_4$ and $R_5$ are not simultaneously hydrogen.

23. A compound according to claim 22 of the formula I, in which $R_1$ is $C_1$—$C_5$alkyl, phenyl, or $C_1$—$C_3$alkyl which is substituted by 1 to 7 fluorine or chlorine atoms, $R_2$ is fluorine, chlorine, bromine,

$C_1$—$C_5$alkyl, $C_1$—$C_5$alkoxy or trifluoromethyl, and $R_3$, $R_4$ and $R_5$ are each independently of one another hydrogen, fluorine, chlorine, bromine, $C_1$—$C_3$alkyl, methoxy or trifluoromethyl.

24. A compound according to any one of claims 21 to 23 of the formula I, in which $R_1$ is methyl.

25. A compound according to any one of claims 21 to 24 of the formula I, in which $R_2$ is chlorine, $R_3$ is hydrogen or chlorine, and $R_4$ and $R_5$ are hydrogen.

26. A compound according to claim 25, of the formula

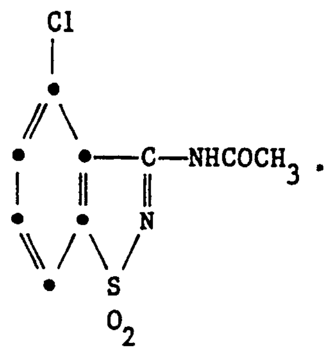

27. A compound according to claim 25, of the formula

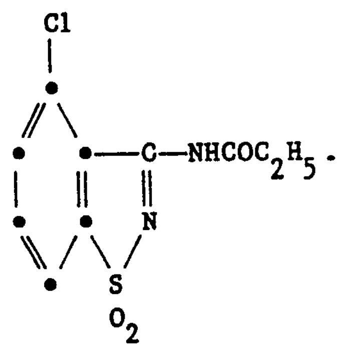

28. A compound according to claim 25, of the formula

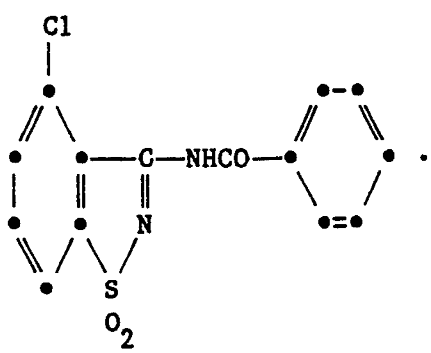

29. A compound according to claim 25, of the formula

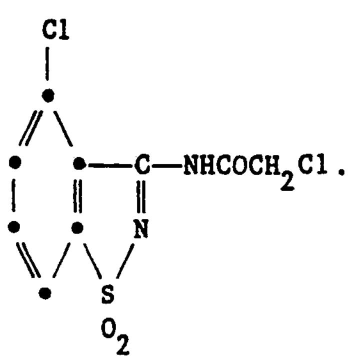

30. A compound according to claim 25, of the formula

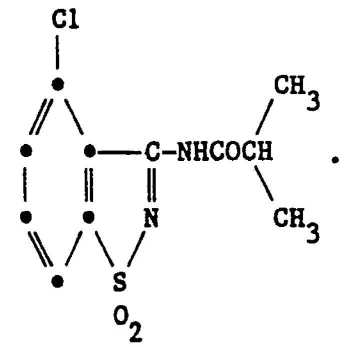

31. A compound according to claim 25, of the formula

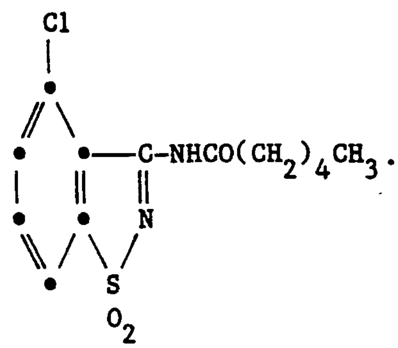

32. A compound according to claim 25, of the formula

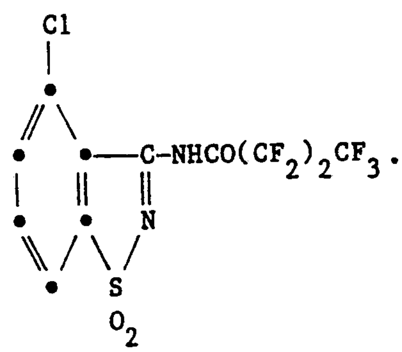

33. A compound according to claim 25, of the formula

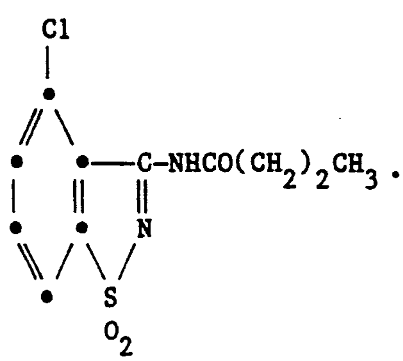

34. A compound according to claim 25, of the formula

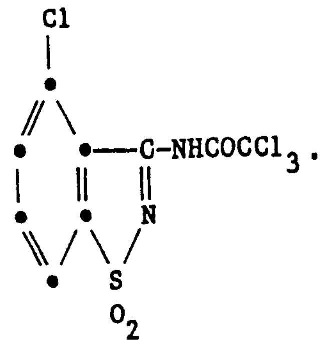

35. A process for the preparation of a compound of the formula I according to any one of claims 21 to 34, which comprises reacting a compound of the formula II

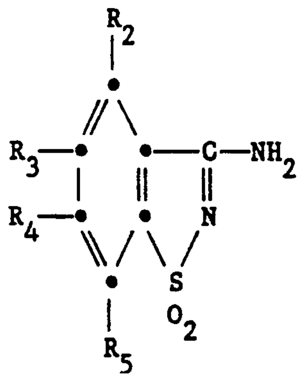

(II)

with a compound of the formula (III)

$$X—COR_1 \qquad (III)$$

or with a compound of the formula IV

$$R_1OC—O—COR_1 \qquad (IV)$$

in which formulae II to IV above the radicals $R_1$ to $R_5$ are as defined in claim 21 for the formula I and X is halogen, preferably chlorine.

36. A pesticidal composition which contains as active component a compound of the formula I according to any one of claims 21 to 34, together with suitable carriers and/or other adjuvants.

37. Use of a compound of the formula I according to claim 21 for controlling pests of plants and animals, in particular insects, representatives of the order Acarina, fungi and bacteria.